(19)

Europäisches
Patentamt
European
Patent Office
Office européen
des brevets

(11) **EP 4 184 526 A1**

(12) **EUROPEAN PATENT APPLICATION**

(43) Date of publication:
**24.05.2023  Bulletin 2023/21**

(21) Application number: **21210045.7**

(22) Date of filing: **23.11.2021**

(51) International Patent Classification (IPC):
*G16H 50/50* (2018.01)

(52) Cooperative Patent Classification (CPC):
**G16H 50/50**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Université d'Aix Marseille**
  **13284 Marseille Cedex 07 (FR)**
• **Institut National de la Santé et de la Recherche Médicale**
  **75013 Paris (FR)**

(72) Inventors:
• **JIRSA, Viktor**
  **13400 Aubagne (FR)**
• **DAINI, Daniele**
  **13005 Marseille (FR)**

(74) Representative: **Macquet, Christophe**
  **Macquet & Associés**
  **Arche des Dolines**
  **7, rue Soutrane**
  **06560 Sophia Antipolis (FR)**

(54) **A METHOD OF SIMULATING A BRAIN NEURAL FIELD**

(57)  The invention relates to a method of simulating a human brain neural field in a computerized platform modelling various zones of a human brain and connectivity between said zones, comprising the following steps: providing the computerized platform modelling the various zones of the human brain and connectivity between said zones; acquiring three-dimensional anatomical structural imaging data of a folded surface of a cortex of a brain of a human patient; personalizing the computerized platform according to the structural data; providing an equation describing a spatiotemporal evolution of the neural field and loading the equation in the computerized platform; performing a projection of the surface of the cortex of the brain of the patient on a spherical surface domain; simulating the neural field in the spherical domain; and translating the simulated neural field in the spherical domain in the cortical domain.

Fig. 13

EP 4 184 526 A1

**Description**

FIELD OF THE INVENTION

The invention relates to a method of simulating a human brain neural field in a computerized platform modelling various zones of a human brain and connectivity between said zones, the method comprising the steps of providing the computerized platform modelling the various zones of the human brain and connectivity between said zones; acquiring three-dimensional anatomical structural imaging data of a folded surface of a cortex of a brain of a human patient; personalizing the computerized platform according to the structural data; and providing an equation describing a spatiotemporal evolution of the neural field and loading the equation in the computerized platform.

BACKGROUND OF THE INVENTION

The Virtual Brain (TVB) is a neuro-informatics platform that provides a realistic connectome-based on brain network models of individual human brains. The Virtual Brain may be used for simulating a human brain-based phenomenon, as epilepsy, as disclosed, for example in the document published on 25 January 2019 under the number WO2018/015778.

In order to simulate a human brain-based phenomenon, that takes place in the brain of an individual, such as the brain of an epileptic patient, structural data of this patient brain are acquired and, in particular, structural data of the cortex of the patient's brain. The Virtual Brain is then personalized according to such data. In the virtual brain, the cortical brain regions are defined, and connected in order to set up the connectome that approximates the brain connectivity between various regions of the patient's brain.

Simulations are then handled on the Virtual Brain personalized according to the patient's brain data.

However, simulating a human brain-based phenomenon does need calculations that are demanding in terms of computational resources. Also, the spatial resolution of the human brain modelized in the Virtual Brain is quite low, practically of about 1 cm$^2$. Such low resolution limits the calculation weight, but also leads to less accurate results in terms of simulations in a wide range of applications, in particular, virtual brain simulations in which scientific or clinical questions are tackled , such as - epilepsy, where building a patient-specific virtual brain allows to identify the excitability spatial distribution through model inversion, - the tumors effects on brain functions, where a brain tumor affects the structure and the activity of the brain, or - brain stimulations, intending to investigate the effects of stimulations such as deep brain stimulation (DBS) and transcranial stimulation with direct/alternate current stimulation (tDCS or tACS) at high-resolution, at the whole-brain level.

Thus, there is a need for developing methods that would increase alternative approaches to increase the spatial resolution of a human brain modelized in the Virtual Brain, that is less demanding in terms of computational resources for simulation, and that thus lead to more accurate simulation results in a wide range of applications such as epilepsy, simulation of tumors effects on brain functions, or brain stimulations.

SUMMARY OF THE INVENTION

Accordingly, a need exists for increasing the spatial resolution of a human brain modelized in the Virtual Brain, that is less demanding in terms of computational resources for simulation, and that thus lead to more accurate simulation results and/or to simulation results that may be obtained faster, for example, 1000 times faster than in the state of the art.

In accordance with a first aspect, the invention concerns a method of simulating a human brain neural field in a computerized platform modelling various zones of a human brain, comprising the following steps:

providing the computerized platform modelling the various zones of the human brain;
acquiring three-dimensional anatomical structural imaging data of a folded surface of a cortex of a brain of a human patient;
personalizing the computerized platform according to the structural data;
providing an equation describing a spatiotemporal evolution of the neural field and loading the equation in the computerized platform;
performing a transformation of the surface of the cortex of the brain of the patient t a spherical surface domain;
simulating the neural field in the spherical surface domain; and
translating the simulated neural field obtained in the spherical domain to the cortical domain.

Preferentially, - the three-dimensional anatomical structural imaging data of the folded surface of the cortex of the brain of the human patient are Magnetic Resonance Imaging data; - the Magnetic Resonance Imaging Data include Diffusion or Functional Magnetic Resonance Imaging Data; - the computerized platform is modelling various zones of the human brain and connectivity between said zones; - the simulation of the neural field in the spherical domain is decomposed into modes using a Fourier transform, and the modes are recomposed using an inverse Fourier transform; - the equation describing the spatiotemporal evolution of the neural field is:

$$\frac{\partial \psi(\Omega_1, t)}{\partial t} = \mathcal{N}(\psi(\Omega_1, t)) +$$

$$+ \int_\Gamma W_{hom}(d_g(\Omega_1, \Omega_2)) S[\psi(\Omega_2, t - d_g(\Omega_1, \Omega_2)/c)] d\Omega_2 +$$

$$+ \int_\Gamma W_{het}(\Omega_1, \Omega_2) S[\psi(\Omega_2, t - d(\Omega_1, \Omega_2)/v)] d\Omega_2$$

wherein

$\Omega$ represents the coordinates of a point on the surfacer $\Gamma$ $\psi(\Omega, t)$ is a vector that contains the state variables of the model,

$W_{hom}(d_g(\Omega_1, \Omega_2))$ is a homogeneous kernel, a function of the geodesic distance between two points that defines a strength and a sign of the local connection,

$W_{het}(\Omega_1, \Omega_2)$ is a heterogeneous kernel, a function that defines if and how any couple of points of the surface is connected through a myelinated connection,

$d(\Omega_1, \Omega_2)$ represents a length of a fibre connecting the two points; - assuming that homogenous connectivity is short-ranged, and that heterogeneous connections are pointwise, the equation describing the spatiotemporal of the neural field is approximated as

$$\frac{\partial \psi(\Omega_1, t)}{\partial t} = -\epsilon \psi(\Omega_1, t) + D\nabla^2 \psi(\Omega_1, t) + \sum_{i,j=1}^{N} \mu_{ij} \delta(\Omega_1 - \Omega_i) S\left(\psi(\Omega_j, t - \frac{d}{v})\right) \quad ; \quad -$$

the equation describing the spatiotemporal evolution of the neural field is linear; - the equation describing the spatiotemporal evolution of the neural field comprises a non-linear term; - the equation describing a spatiotemporal evolution of the neural field is calculated using a finite volume method; - the surface of the cortex of the brain of the patient is tessellated according to a mesh of at least 10000 vertices; - the human brain is an epileptic human brain, and wherein the simulated neural field is the neural field of the epileptic human brain during an epileptic seizure; - the human brain is a human brain including a tumour, and wherein effects of the tumour on a structure and/or an activity of the human brain are simulated; - effects of stimulation, such as deep brain stimulation or transcranial stimulation are simulated; and - the human brain is an Alzheimer human brain, and wherein the simulated neural field is the neural field of the Alzheimer human brain.

According to a second aspect, the invention concerns a simulator of a human brain neural field in a computerized platform modelling various zones of a human brain, comprising:

the computerized platform modelling the various zones of the human brain;
three-dimensional anatomical structural imaging data of a folded surface of a cortex of a brain of a human patient;
the computerized platform being personalized according to the structural data;
an equation describing a spatiotemporal evolution of the neural field, the equation being loaded in the computerized platform;
computing means for performing a transformation of the surface of the cortex of the brain of the patient to a spherical surface domain;
simulating computerized means for simulating the neural field in the spherical surface domain; and
translating computerized means for translating the simulated neural field obtained in the spherical domain to the cortical domain.

According to a third aspect, the invention concerns a machine-readable medium, having instructions stored thereon, for simulating a human brain neural field in a computerized platform modelling various zones of a human brain, comprising the following steps:

> providing the computerized platform modelling the various zones of the human brain;
> acquiring three-dimensional anatomical structural imaging data of a folded surface of a cortex of a brain of a human patient;
> personalizing the computerized platform according to the structural data;
> providing an equation describing a spatiotemporal evolution of the neural field and loading the equation in the computerized platform;
> performing a transformation of the surface of the cortex of the brain of the patient t a spherical surface domain;
> simulating the neural field in the spherical surface domain; and
> translating the simulated neural field obtained in the spherical domain to the cortical domain.

According to a fourth aspect, the invention concerns a use of data of a brain of a patient for the manufacture of a simulator of a human brain neural field in a computerized platform modelling various zones of a human brain, comprising:

> providing the computerized platform modelling the various zones of the human brain;
> acquiring three-dimensional anatomical structural imaging data of a folded surface of a cortex of a brain of a human patient;
> personalizing the computerized platform according to the structural data;
> providing an equation describing a spatiotemporal evolution of the neural field and loading the equation in the computerized platform;
> performing a transformation of the surface of the cortex of the brain of the patient t a spherical surface domain;
> simulating the neural field in the spherical surface domain; and
> translating the simulated neural field obtained in the spherical domain to the cortical domain.

BRIEF DESCRIPTION OF THE DRAWINGS

Other features and aspects of the present invention will be apparent from the following description and the accompanying drawings, in which

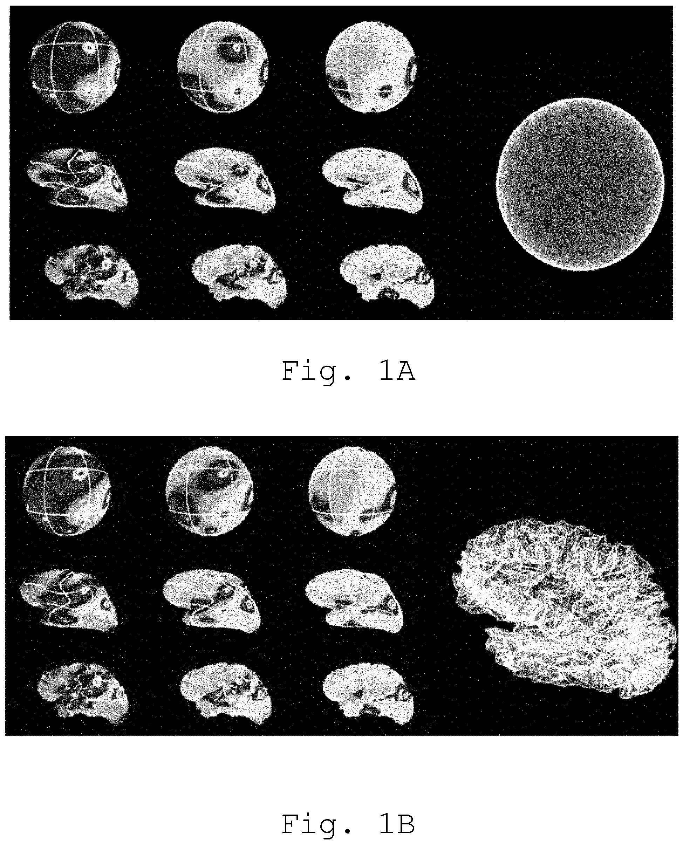

> Figs. 1A and 1B is a comparison between the spatiotemporal patterns of the neural field obtained numerically solving an equation (1) mentioned later in the description of the invention on the spherical surface (Fig. 1A) and on the folded one (Fig. 1B). In both cases, the solution is represented at three different times, reading from left to right, over three different surfaces: the folded, the inflated and the spherical surface. While a slight deformation is visible, the overall spatiotemporal evolution is preserved, as confirmed by the 94% goodness of fit obtained with a mode level cognitive subtraction analysis. In this simulation, the parameters have been chosen to produce an oscillatory state; the neural field falls into a limit cycle.

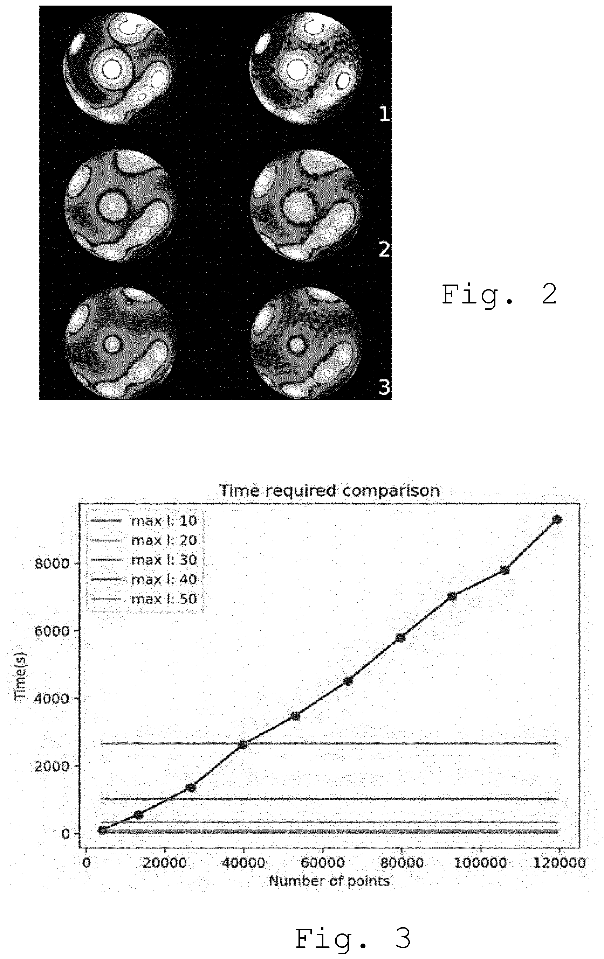

> Fig. 2 is 3D representation of the neural field calculated on the sphere using a 34x34 connectivity matrix ($\mu_{i,j}$ in equation (1)). The surface has been built with 132608 vertices. The time evolves from top to bottom, and the parameters of the system have been chosen so that the activity oscillates in a periodic pattern (limit cycle). On the left, the Finite Volume Method (FVM) simulation is shown, on the right the Mode Decomposition (MD) with maximum 1 = 50.
> Fig. 3 illustrates the wall time (in seconds) of the FVM simulations that is shown in red dots for different number of neural populations defining the surface; as a comparison, the time needed for MD simulations with different truncation values are plotted as horizontal lines. A fully implicit Crank-Nicolson scheme has been used for both the FVM and the MD simulations. Only the time consumption of the principal loop of the simulation is considered.

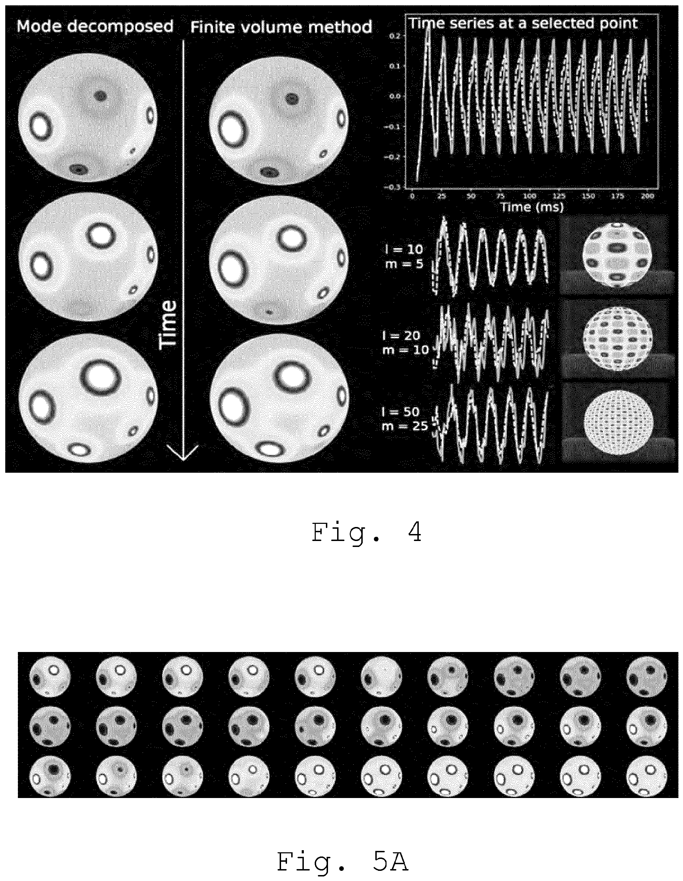

> Fig. 4 illustrates, on the left, three frames from the FVM and the Spherical Harmonics Transform (SHT) simulation, to show the spatiotemporal representation of the neural field. On the right-top corner, the time series at a selected point is represented for both the FVM (solid light dark line) and the SHT simulation (dashed light grey line) . On the bottom-right corner, the time series obtained projecting the neural field on three different spherical harmonics ($Y_{10,5}$, $Y_{20,10}$, $Y_{50,25}$) are represented; for visualization purposes, the time series have been shown only for the first 4000 time-steps, and have been rescaled in amplitude.

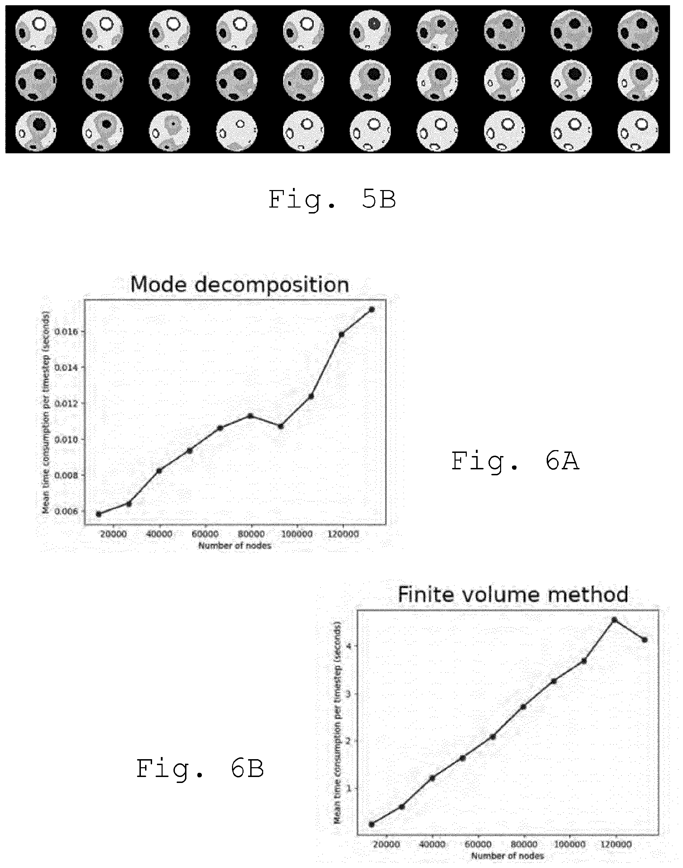

> Figs. 5A and 5B are spatiotemporal representations of the FVM simulation (Fig. 5A) and the spherical harmonic transform for numerical simulation (shtns) (Fig. 5B). The time increase from left to right, from top to bottom.
> Fig. 6A and 6B illustrate the execution time and its dependency on the size of the mesh. The time consumption has been estimated as the minimum wall time over 3 runs. The simulation has been run for 4000 timesteps using a buffer, the vertical axis shows the average time consumption for a single time-stepping operation. The different surfaces have been obtained from the same human patient, using the FreeSurfer™ decimate function.

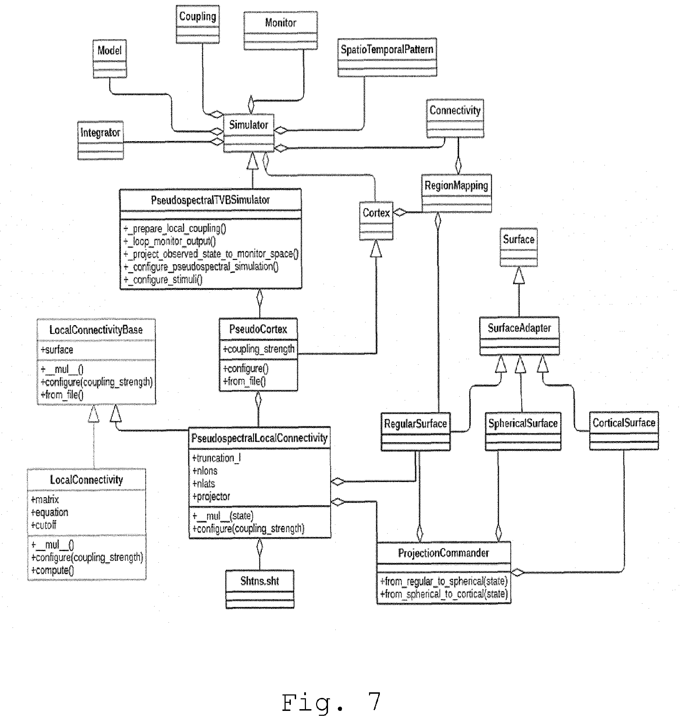

Fig. 7 is a class diagram illustrating the implementation of the pseudo-spectral simulator. The Integrator, Model, coupling, Monitor, Spatiotemporal Pattern, Connectivity, Simulator, Cortex, Region Mapping, Surface objects represent classes taken from the TVB library; in pale lines, the relations that have been overridden into the pseudo-spectral version of the simulator. Not all the objects or relationships are represented into this diagram.

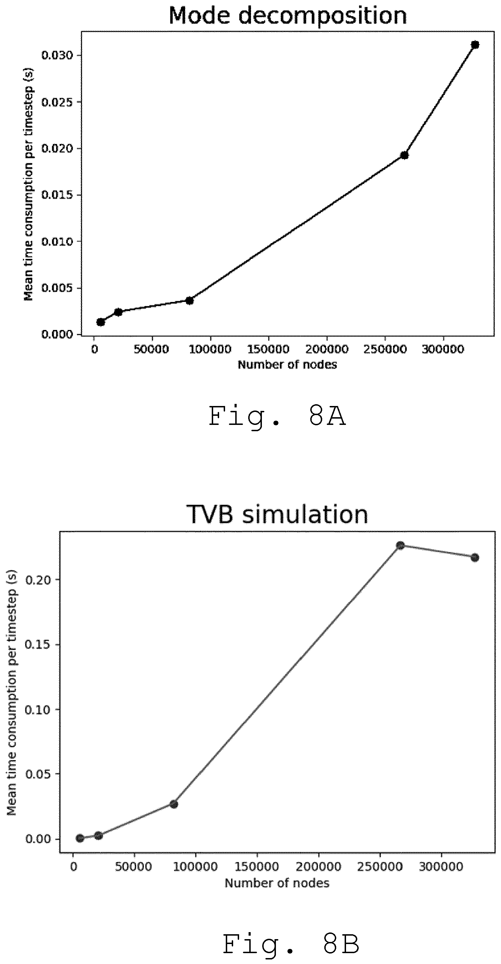

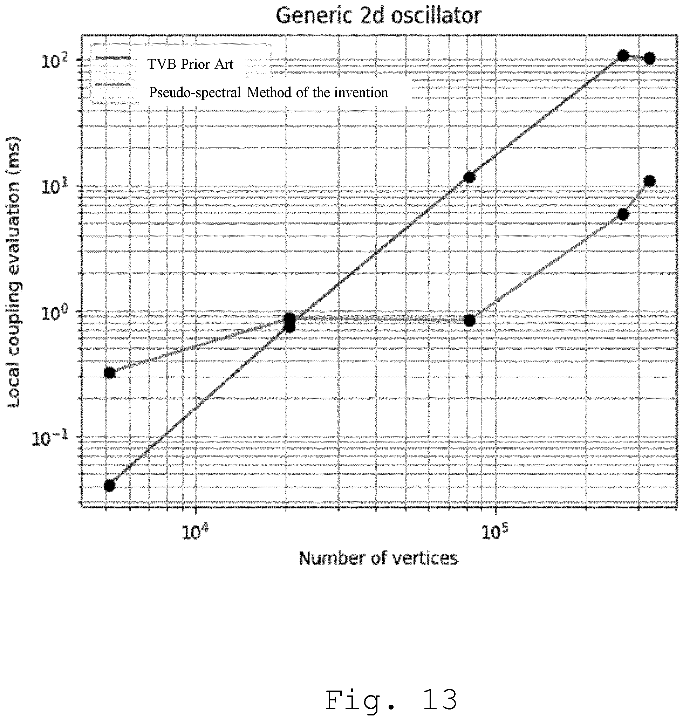

Figs. 8A and 8B illustrate average wall time for a single time-stepping operation; to be more precise, this represents the average time spent in a call of the scheme method of the integrator used. Using the same profiling data, a comparison of the time spent in the evaluation of the local coupling term is shown in Fig. 13.

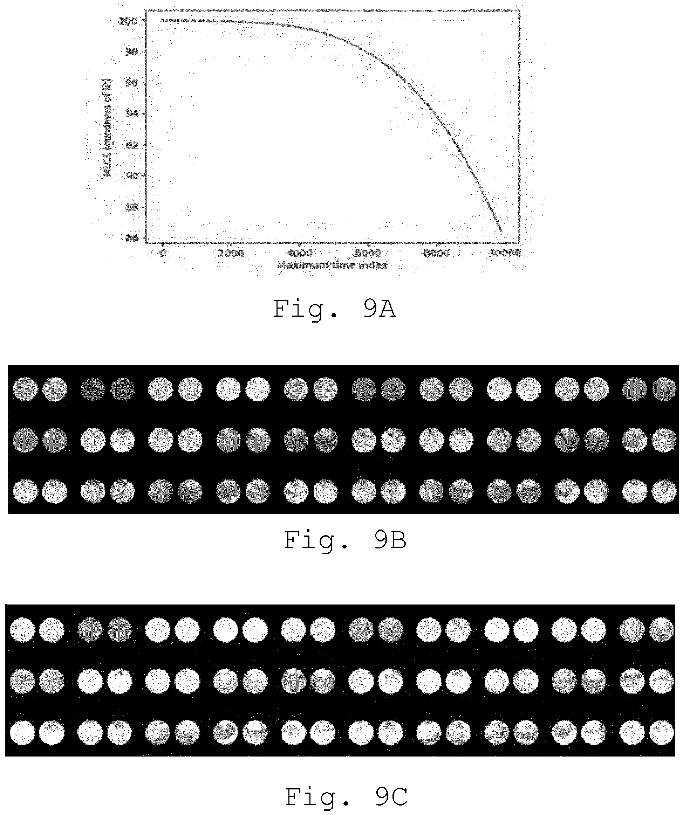

Fig. 9A illustrates the Mode Level Cognitive Subtraction (MLCS) estimated at every timestep using the Principal Component Analysis (PCA) technique. Fig. 9B is a spatiotemporal representation of the pseudo-spectral simulation (time evolves from left to right, from top to bottom. Only the left hemisphere is shown). Fig. 9C is a spatiotemporal representation of the Laplace-Beltrami simulation.

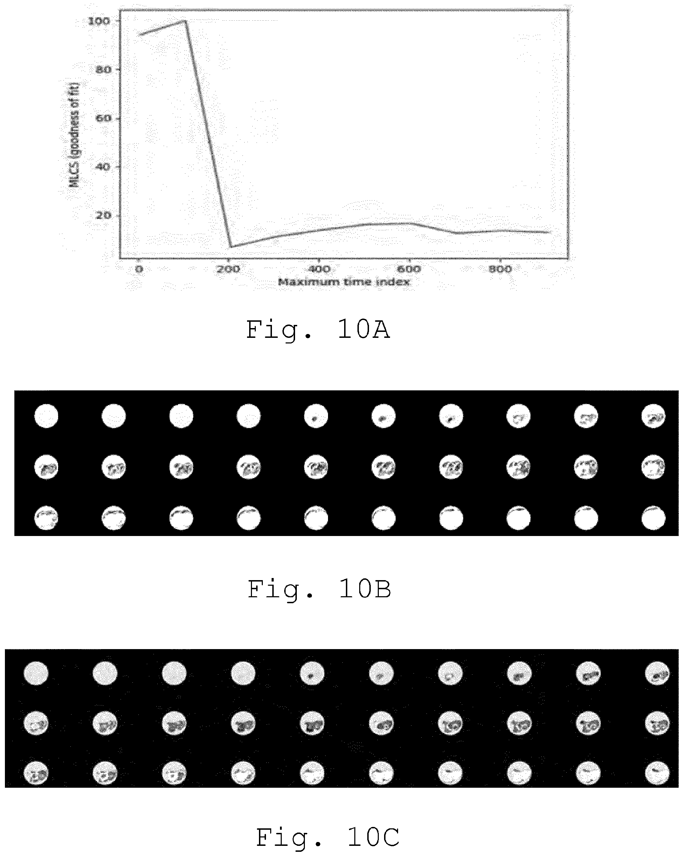

Figs. 10A, 10B and 10C illustrate a simulation of a seizure: in Fig. 10A, the MLCS is estimated at every timestep using the PCA technique. It quickly drops with the seizure onset, indicating that the pseudo-spectral simulation is recruiting different variance modes; Fig. 10B is a spatiotemporal representation of the pseudo-spectral simulation (time evolves from left to right, from top to bottom. Only the left hemisphere is shown); Fig. 10C is a representation of the TVB simulation.

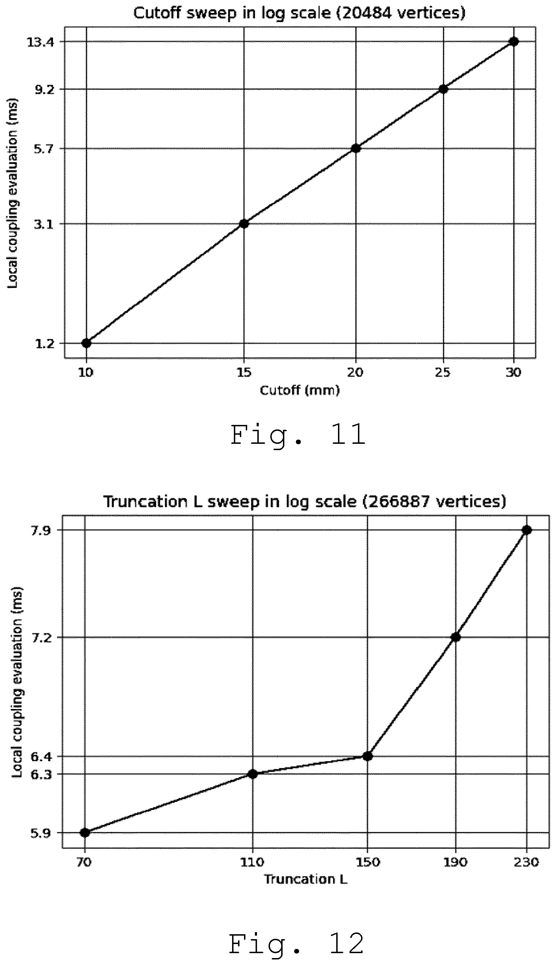

Fig. 11 illustrates a performance evaluation using a mesh with 20484 vertices using a Gaussian kernel as in equation (20). The plot is represented using a logarithmic scale.

Fig. 12 illustrates a performance evaluation using a mesh with 266887 vertices using the pseudo-spectral approach. The scaling of the computational burden with the number of modes required is shown. The plot is represented using a logarithmic scale.

Fig. 13 represents, in dark grey, the time consumption of the average of a single local coupling evaluation for a TVB simulation according to the prior art, with cut-off set to 10 mm. In light grey, the analogous is shown using the pseudo-spectral approach truncating the modes at L = 70. It appears that the pseudo-spectral method performs better of at least an order of magnitude respect to the TVB simulation according to the prior art. If the number of vertices is small, for example under 10000, the TVB simulation according to the prior art performs comparably than the pseudo-spectral method counterpart according to the invention; it is noticed that, in the case of 5000 vertices, the pseudo-spectral approach computes the Fast Fourier Transforms (FFT) over a grid of 10000 points to satisfy the constrain of the 70 modes needed. Sparse meshes are of interest for the application of the method, but of a limited interest.

## DETAILED DESCRIPTION OF THE INVENTION

The method according to the invention comprises various steps that are computer implemented. A computer readable medium is encoded with computer readable instructions for performing the steps of the method according to the invention.

It comprises a step of providing a computerized model modelling various regions of a primate brain, in particular a human brain, and connectivity between said regions. This brain is a virtual brain. It is a neuro-informatics platform for full brain network simulations using biologically realistic connectivity. This simulation environment enables the model-based inference of neurophysiological mechanisms across different brain scales that underlie the generation of macroscopic neuroimaging signals including functional Magnetic Resonance Imaging (fMRI), EEG and Magnetoencephalography (MEG). It allows the reproduction and evaluation of personalized configurations of the brain by using individual subject data.

It further comprises a step of providing said computerized model with a model able to reproduce an epileptic seizure dynamic in the primate brain, said model being a function of a parameter that is the epileptogenicity of a region of the brain.

Preferentially, the model able to reproduce the epileptic seizure dynamic in the human brain is a model which reproduces the dynamics of an onset, a progression and an offset seizure events, that comprises state variables coupling two oscillatory dynamical systems on three different timescales, a fastest timescale wherein state variables account for fast discharges during an ictal seizure state, an intermediate timescale wherein state variables represent slow spike-and-wave oscillation, and a slowest timescale wherein a state variable is responsible for the transition between interictal and ictal states, and wherein a degree of epileptogenicity of a region of the brain is represented through a value of an excitability parameter.

Also, the invention comprises a step of providing structural data of the epileptic patient brain and personalizing the computerized model using said structural data in order to obtain a virtual epileptic patient (VEP) brain model. The structural data are, for example, images data of the patient brain acquired using magnetic resonance imaging (MRI), diffusion-weighted magnetic resonance imaging (DW-MRI), nuclear magnetic resonance imaging (NMRI), or magnetic resonance tomography (MRT). Preferentially, the structural data of the epileptic patient brain comprise non-invasive T1-weighted imaging data and/or diffusion MRI images data.

Such a model is disclosed, for example, in the publication document entitled "On the nature of seizure dynamics", Jirsa et al., Brain 2014, 137, 2210-2230, which is incorporated herein, by citation of reference. This model is named "Epileptor". It comprises five state variables acting on three different time scales. On the fastest time scale, state variables $x_1$ and $y_1$ account for the fast discharges during the seizure. On the slowest time scale, the permittivity state variable z accounts for slow processes such as variation in extracellular ion concentrations, energy consumption, and tissue oxygenation. The system exhibits fast oscillations during the ictal state through the variables $x_1$ and $y_1$. Autonomous switching between interictal and ictal states is realized via the permittivity variable z through saddle-node and homoclinic bifurcation mechanisms for the seizure onset and offset, respectively. The switching is accompanied by a direct current (DC) shift, which has been recorded in vitro and in vivo. On the intermediate time scale, state variables $x_2$ and $y_2$ describe the spike-and-wave electrographic patterns observed during the seizure, as well as the interictal and preictal spikes when excited by the fastest system via the coupling $g(x_1)$. The equations of the model read as follows:

$$\dot{x}_1 = y_1 - f_1(x_1, x_2) - z + I_1$$

$$\dot{y}_1 = 1 - 5x_1^2 - y_1$$

$$\dot{z} = \frac{1}{\tau_0}(4(x_1 - x_0) - z)$$

$$\dot{x}_2 = -y_2 + x_2 - x_2^3 + I_2 + 0.002g(x_1) - 0.3(z - 3.5)$$

$$\dot{y}_2 = \frac{1}{\tau_2}(-y_2 + f_2(x_1, x_2))$$

where

$$f_1(x_1, x_2) = \begin{cases} x_1^3 - 3x_1^2 & \text{if } x_1 < 0 \\ \left(x_2 - 0.6(z-4)^2\right)x_1 & \text{if } x_1 \geq 0 \end{cases}$$

$$f_2(x_1, x_2) = \begin{cases} 0 & \text{if } x_2 < -0.25 \\ 6(x_2 + 0.25)x_1 & \text{if } x_2 \geq -0.25 \end{cases}$$

$$g(x_1) = \int_{t_0}^{t} e^{-\gamma(t-\tau)} x_1(\tau) d\tau$$

and $x_0$ = -1.6; $\tau_0$ = 2857; $\tau_2$ = 10; $I_1$ = 3.1; $I_2$ = 0.45; $\gamma$ = 0.01. The parameter $x_0$ controls the tissue excitability, and is epileptogenic triggering seizures autonomously, if $x_0$ is greater than a critical value, $x_{0C}$ = -2.05. Otherwise, the tissue

is healthy. $I_1$ and $I_2$ are passive currents setting the operating point of the model. The model of the propagation zone is identical to the one of an EZ, however with an excitability parameter inferior to the critical value $x_{0C}$ = -2.05. All other brain areas may be modelled by Epileptors with excitability values far from the threshold, or equivalently standard neural population models as disclosed in Paula Sanz Leon et al., 11 June 2013, which is incorporated herein, by citation of reference. The coupling between brain areas follows a mathematical model as disclosed in the publication document entitled "Permittivity Coupling across Brain Regions Determines Seizure Recruitment in Partial Epilepsy", Timothee Proix et al., The Journal of Neuroscience, November 5, 2014, 34(45): 15009-15021, which is incorporated herein, by citation of reference.

Permittivity coupling quantifies the influence of neuronal fast discharges $x_{1j}$ of a remote region j on the local slow permittivity variable of a region i. Changes in ion homeostasis are influenced by both local and remote neuronal discharges via a linear difference coupling function, which quantifies the deviation from the interictal stable state as a perturbation perpendicular to the synchronization manifold. The linearity is justified as a first order approximation of the Taylor expansion around the synchronized solution. Permittivity coupling further includes the connectome $C_{ij}$, a scaling factor G, which both are absorbed in $K_{ij} = GC_{ij}$. The permittivity coupling from area j to area i reads

$$\sum_{j=1}^{N} K_{ij} \cdot \left( x_{1,j}(t - \tau_{ij}) - x_{1,i}(t) \right)$$

, where $\tau_{ij}$ denotes the signal transmission delay. When loading the models of the epileptogenic zone (EZ) and propagation zone (PZ) in the virtual brain, the signal transmission time delays are here neglected, because synchronization effects will not be considered, but rather only the epileptic spread, which is determined by the slow dynamics of the permittivity coupling. Mathematically, the virtual brain then corresponds to the following equations:

$$\dot{x}_{1,i} = y_{1,i} - f_1\left(x_{1,i}, x_{2,i}\right) - z_i + I_{1,i}$$

$$\dot{y}_{1,i} = 1 - 5\left(x_{1,i}\right)^2 - y_{1,i}$$

$$\dot{z}_i = \frac{1}{\tau_0}\left( 4(x_1 - x_0) - z_i - \sum_{j=1}^{N} K_{ij} \cdot \left( x_{1,j} - x_{1,i} \right) \right)$$

$$\dot{x}_{2,i} = -y_{2,i} + x_{2,i} - \left(x_{2,i}\right)^3 + I_{2,i} + 0.002g\left(x_{1,i}\right) - 0.3\left(z_i - 3.5\right)$$

$$\dot{y}_{2,i} = \frac{1}{\tau_2}\left(-y_{2,i} + f_2\left(x_{1,i}, x_{2,i}\right)\right)$$

The invention concerns a new, efficient algorithm to numerically simulate neural field equations of the form:

$$\frac{\partial \psi(\Omega_1, t)}{\partial t} = \mathcal{N}(\psi(\Omega_1, t)) +$$

$$+ \int_\Gamma W_{hom}(d_g(\Omega_1, \Omega_2)) S[\psi(\Omega_2, t - d_g(\Omega_1, \Omega_2)/c)] d\Omega_2 +$$

$$+ \int_\Gamma W_{het}(\Omega_1, \Omega_2) S[\psi(\Omega_2, t - d(\Omega_1, \Omega_2)/v)] d\Omega_2$$

where, on the right-hand side (RHS) the first term represents the dynamical evolution of an isolated neural population; the second term represent the homogeneous part of the connectivity, that models the short-ranged local connections around a neural population; the third and last term represent the heterogeneous part of the connectivity, that models the white matter connections, and

$\Omega$ represents the coordinates of a point on the $\Gamma$ surface,
$\psi(\Omega, t)$ is a vector that contains the state variables of the model.
$W_{hom}(d_g(\Omega_1, \Omega_2))$ is the homogeneous kernel, a function of the geodesic distance between two points that defines the strength (and the sign) of the local connections,
$W_{het}(\Omega_1, \Omega_2)$ is the heterogeneous kernel, a function that defines if and how any couple of points of the surface is connected through a myelinated connection,
$d(\Omega_1, \Omega_2)$ represents the length of the fibre connecting the two points.

According to the invention, it is illustrated the idea of the mode decomposition (MD) technique. Particularly simple equations are chosen to represent the spatiotemporal evolution of the neural field, and the conceptual steps of the algorithm are shown.

Numerical methods needed to tackle nonlinear neural field equations are illustrated. A benchmark of the techniques is included, together with the benchmark of an implementation of a neural field simulator based on the finite volume method, for comparison.

Also, a comparison of the method according to the invention with a state-of-the-art neural field simulator is described. It is shows that the mode decomposition technique according to the invention is one order of magnitude less computationally expensive than the state of the art TVB approach. Two use cases have been selected to represent the loss of accuracy that the technique introduces. According to the method of the invention, the cortical surface of the brain of the patient is tessellated according to a mesh of vertices, whatever the number of vertices. Preferentially, the number of vertices is of at least 5000. More preferentially, it of at least 10000. For example, it is comprised between approximately 100000 and approximately 500000 vertices.

Spectral approach: a toy model case

In this section, the method using a particularly simple toy model is applied to illustrate a concrete application of the invention. It is noted that this does not represent the most generic application of the invention. The neural field equation considered here is linear in absence of connections, so that the decomposition into modes can be performed analytically. Instead of mapping the equation to a set of delayed differential equations with as many elements as the nodes of the mesh, as it would happen with a finite element or volume method, here the neural field is decomposed into spatial modes. Every spatial mode has a weight in determining the whole solution; such a weight evolve in time following an equation that can be derived from the starting neural field equation. Thus, the number of equations depends on the number of spatial modes that are considered. This approach is typically referred to as a spectral technique.

In the following, we will suppose that the spatial domain of the neural field is a sphere. In practice, the cortical surface is not spherical but there are techniques (that are implemented as routines or pipelines for example in Freesurfer™) that inflate the cortical sheet of every hemisphere into a sphere; the edges of the mesh describing the folded surface undergo an unavoidable deformation, but this perturbation is minimized by the algorithm and, for a dense enough mesh, it does not disrupt the dynamics (see Fig. 1). Thus, it is possible to solve the problem on the spherical surface, and then map it back to the folded surface to estimate the source signal.

Solving the neural field equations on the mode space proved to be very efficient: the necessary number of spherical harmonics to obtain a consistent spatial resolution is small enough, in our use case, to get a high speed up in the simulations and to drastically reduce the memory consumption. More interestingly, the complexity of the resulting algorithm does not depend on the resolution of the mesh, as the neural field is computed in the mode space; this won't be the case for generic neural field equations, as we will show in the next sections.

As an example, the mode decomposition technique has been applied to the following delayed integro-differential equation, describing the temporal and spatial evolution of a neural field, where the local node dynamics is linear:

$$\frac{\partial \psi(\Omega_1, t)}{\partial t} = - \epsilon \psi(\Omega_1, t) +$$

$$+ \int_\Gamma W_{hom}(d_g(\Omega_1, \Omega_2)) S[\psi(\Omega_2, t - d_g(\Omega_1, \Omega_2)/c)] d\Omega_2 +$$

$$+ \int_\Gamma W_{het}(\Omega_1, \Omega_2) S[\psi(\Omega_2, t - d_h(\Omega_1, \Omega_2)/v)] d\Omega_2$$

Assuming that the homogeneous connectivity is short-ranged, and that the heterogeneous connections are pointwise, the previous equation can be approximated as in reference document [3]:

$$\frac{\partial \psi(\Omega_1, t)}{\partial t} = -\epsilon \psi(\Omega_1, t) + D \nabla^2 \psi(\Omega_1, t) + \sum_{i,j=1}^{N} \mu_{ij} \delta(\Omega_1 - \Omega_i) S \left( \psi(\Omega_j, t - \frac{d}{v}) \right) \qquad (1)$$

Here, the second term, describing the local connectivity, is reduced to a Laplacian operator applied to the state vector; the third and last term represents the white matter 20 as point-to-point connections. $\delta(\Omega_1 - \Omega_i)$ is the Dirac Delta.

In order to apply the mode decomposition, the neural field is decomposed through the following approach:

$$\psi(\Omega, t) = \sum_{l,m}^{\infty} Y_{l,m}(\Omega) \xi_{l,m}(t) \qquad (2)$$

And, after some algebra, the delayed differential equations for the coefficients are obtained:

$$\left( \frac{\partial}{\partial t} + \epsilon \right) \xi_{l,m}(t) = - D(l(l+1)) \xi_{l,m}(t) + \sum_{i,j=1}^{N} \mu_{i,j} \sum_{l',m'} Y_{l,m}^\dagger(\Omega_i) S \left( Y_{l',m'}(\Omega_j) \xi_{l',m'} \left( t - \frac{d_{i,j}}{v} \right) \right) \qquad (3)$$

The diffusive term is damping the coefficients corresponding to higher 1 values; this suggests that a truncation of the series of equation 2 to a summation with a finite number of terms L might be a viable approximation of the series itself. The simulation is thus carried on this reduced system, and the spatial dependency is recovered at the end, with the desired resolution, through the truncated version of equation (2).

A direct evaluation of the solution in equation (1) has been calculated through the FVM. The surface has been tessellated into small areas (one per vertex) $A_i \in S^2$, the $\psi$ has been evaluated as an average in a small area around every vertex of the mesh:

$$\psi_i(t) = \frac{1}{m(A_i)} \int_{A_i} \psi(\Omega, t) \, dA \qquad (4)$$

$$\dot{\psi}_i(t) = -\epsilon \psi_i(t) + \frac{D}{m(A_i)} \int_{A_i} \Delta \psi(\Omega, t) dA + \frac{1}{m(A_i)} \sum_{ij} \mu_{ij} S(\psi(\Omega_j, t - \tau_{ij})) \qquad (5)$$

Where the diffusive term has been approximated according to reference document [4].

$$\frac{D}{m(A_i)} \int_{A_i} \Delta\psi(\Omega,t)dA \simeq \frac{D}{m(A_i)} \sum_{i_j \in \chi_i} p_{i,i_j}(\psi_i(t) - \psi_{i_j}(t)) \qquad (6)$$

### 1.1 Comparison

A visual representation of the spatiotemporal evolution simulated with the two methods is shown in Fig. 2.

The speed up obtained is remarkable. In Fig. 3, the time needed for a FVM simulation as a function of the number of neural populations that constitutes the surface is shown. The parameters of equation (1) have been chosen to produce an oscillatory activity. Notice that the mode decomposition does not depend at all on the number of nodes of the mesh, as the entire simulation is performed in the mode space. This will not be the case with models that involve a non-linear term. In that case, the non-linearity is expressed in the mode space through a tensor that couples different modes; such tensor contains so many elements that it imposes a memory constrain, and the spectral method becomes more computationally expensive then the FVM.

### The pseudo-spectral method

In the previous section we have applied the mode decomposition to a toy model case. While the results were promising, the technique has to be modified to apply it for more meaningful use cases. Here we present a more general method adding a generic non-linear term to equation (1) .

The pseudo-spectral method is well known in applied mathematics and scientific computing, and it is typically used for the solution of partial differential equations with non-linear terms. Let $\Omega = (\theta, \phi)$ describe the position over the spherical surface representing a single hemisphere.

The next steps do not constrain the method according to the invention, but make it concrete.

The equation describing the spatiotemporal evolution of the neural field that we are interested into is the following:

$$\frac{\partial\psi(\Omega_1,t)}{\partial t} = -\epsilon\psi(\Omega_1,t) + N(\psi(\Omega_1,t)) + D\nabla^2\psi(\Omega_1,t) + \sum_{i,j=1}^{N} \mu_{ij}\delta(\Omega_1 - \Omega_i)S\left(\psi\left(\Omega_j, t - \frac{d_{ij}}{v}\right)\right) \qquad (7)$$

Wherein $N(\psi(\Omega_1, t))$ is a non-linear function of $\psi(\Omega_1, t)$ and $d_{1j}$ is the length of the heterogeneous connection between the point in $\Omega_1$ and the point in $\Omega_j$.

To numerically solve the equation (7) we need to discretize the spatial domain; as we have done above in connection with equation (5), the finite volume method is applied.

$$\dot{\psi}_i(t) = -\epsilon\psi_i(t) + N(\psi_i(t)) + D\nabla^2\psi_i(\Omega,t) + \frac{1}{m(A_i)}\sum_{ij}\mu_{ij}S(\psi(\Omega_j, t - \tau_{ij})) \qquad (8)$$

The time is discretized with a semi-implicit Euler method and with some algebraic manipulations it is found the equation that allows to determine the value of the neural field and the next time step n + 1 knowing its value at the time step n:

$$\left(\frac{1}{\Delta t} + \epsilon - D\nabla^2\right)\psi_i^{n+1} = \frac{\psi_i^n}{\Delta t} + N(\psi_i^n) + \sum\mu_{ij}S\left(\psi_j^{n+1-\tau_{ij}}\right) \qquad (9)$$

Now, a spherical harmonics transform (SHT) is applied on both sides, and its linearity property is used on the left side. It is found:

$$SHT(\psi_i^{n+1}) = \frac{SHT\left(\frac{\psi_i^n}{\Delta t} + N(\psi_i^n) + \sum\mu_{ij}S\left(\psi_j^{n+1-\tau_{ij}}\right)\right)}{\frac{1}{\Delta t} + \epsilon + Dl(l+1)} \qquad (10)$$

Thus, applying an anti-transform on both sides, the value of $\psi_i^{n+1}$ is determined. So, at every time step, a spherical harmonics transform is run on the numerator of the RHS, and then an anti-transform of the whole RHS provides the value of the neural field at the next time step. In order to be efficient, a fast spherical harmonics transform is necessary: the SHTns library, based on reference document [5], contains an implementation of the Gauss Legendre algorithm, that is a fast spherical harmonics transform on a Gauss-Legendre grid.

Comparison

The spatial domain has been discretized using data obtained with an inflation of the surface of a hemisphere of a real human brain cortex. The purpose of this method is to estimate the neural field over the data points on the sphere so that the neural field can be projected back on the folded surface; thus, the coordinates of the nodes representing the spherical surface are fixed by the data. But the spherical harmonics transform efficiency strongly depends on the sampling of the surface; we have tried to perform it directly on the data using a different library , based on reference document [7], but the results were not satisfying. The time consumption was comparable to what was obtained with a direct evaluation of the neural field, if not worse.

Here are presented the results obtained mapping the spherical surface of the data to a regular (Gauss-Legendre) grid on the sphere. Every point is mapped to the nearest one on the regular grid (thus, this is not a one-to-one mapping), and the systematically error induced by this is simply accepted. The results obtained are compared using equation (10) to the ones obtained solving directly equation (5). For simplicity, we will call the first is called a shtns simulation and the second one a direct simulation.

In the following, the non-linear term has been supposed to be cubic $N(\psi) = k\psi^3$. Figure 5 represent the neural field simulated in the case of 34 heterogeneous connections, with an anti-symmetric coupling strength matrix. The surface of the sphere has been sampled with 92826 points. The regular grid has been built so that the total number of points remain the same, with 162 points along the polar angle and 573 on the azimuth. Modifying the choice of the regular grid sampling affects the accuracy of the method.

In the shtns simulation the mode decomposition is truncated to 1 = 50. In Fig. 4 a three-dimensional visualization of the spatio-temporal patterns is represented, while in Fig. 5 more-time snapshots are shown.

While some accuracy is lost, the pseudo-spectral method captures most of the variance of the control simulation. On the performance side, computing 20 ms of neural field evolution requires around 20 seconds of computation, while the direct simulation needs around 30 minutes in the 92826 points case. The dependence of the time for an 80 ms simulation to the number of vertices describing the neural sheet is shown in Fig. 6.

Pseudo-spectral method compared to The Virtual Brain

In the previous section, we have reported the comparison between the pseudo-spectral simulator and an implementation of a neural field simulator using the finite volume method. To validate further the pseudo-spectral approach and obtain more reliable benchmarking results, the pseudo-spectral method has been implemented in The Virtual Brain (TVB) framework (see Fig. 7). All the benchmarking results in this section have been obtained using the standard library of Python Cprofile, and a laptop with the processor Intel(R) Core(TM) i9-10885H CPU @ 2.40GHz. Only single-threaded simulations have been run: while in the pseudo-spectral case the library that performs the FFT supports parallelization, to the knowledge of the author there is not such an equivalent for sparse matrix vector multiplication.

TVB allows for two different types of simulations: region-based and surface-based. Here, we are interested into the surface based, so that we will refer to TVB surface-based simulation simply as TVB simulations.

TVB simulations provide a tool to simulate a neural field through a brain network model (BNM) approach; to do so, every vertex of the mesh is considered as a neural mass.

The bottleneck of these simulations is constituted by the evaluation of the homogeneous coupling term:

$$I = \int_{S^2} W_{hom}(d(\Omega, \Omega^*))\psi(\Omega^*, t)d\Omega^* \qquad (11)$$

where $\psi$ is the state variable (typically a vector). In a TVB simulation, such term is computed as a matrix times vector product:

$$I \simeq \sum_j W_{ij}\psi_j \qquad (12)$$

The homogeneous kernel $W_{hom}$ is typically a short-ranged function of the distance between vertices, thus the matrix $W_{ij}$ is a sparse matrix. In TVB, this multiplication is performed using the Scipy™ library (https://www.scipy.org/).

The Laplacian operator approximation

The pseudo-spectral simulator implements an alternative approach to compute this term. As it can be seen in Fig. 7, the pseudo-spectral simulator extends the TVB simulator by modifying only the cortex object, that is the object responsible of building the elements needed to compute equation (12). Instead of computing the matrix vector product, the pseudo-spectral method maps the problem into the modes space using the Fast Fourier Transform (FFT). At the moment, the implementation is computing an approximated version of equation (11), that involves the Laplacian operator. It can be shown that equation (11) is fully equivalent to:

$$I = \sum_{l,m}^{\infty} \bar{\psi}_{l,m}(t) \sqrt{\frac{4\pi}{2l+1}} Y_{l,m}(\Omega) \int_{S^2} W_{hom}(\overline{\Omega}) Y_{l,0}(\overline{\Omega}) \, d\overline{\Omega} \qquad (13)$$

where the $\psi$ has been expanded into spherical harmonics and a rotation has been performed in the integral to simplify the expression of the distance. Equation (13) is exact; we now introduce the first approximation. Considering that the homogeneous kernel is short ranged, the integrand function is reasonably different from zero only in a short area around the north pole. Thus, we approximate the function $Y_{1,0}$ with a Taylor expansion (we stop at the second order approximation). Using the symmetry properties of spherical harmonics, it is found:

$$I \simeq \left( \int_{S^2} W_{hom}(\overline{\Omega}) d\overline{\Omega} \right) \psi(\Omega,t) - \sum_{l,m} l(l+1) \bar{\psi}_{l,m} Y_{l,m}(\Omega) \frac{1}{4} \int_{S^2} \bar{\theta}^2 W_{hom}(\overline{\Omega}) d\overline{\Omega} = , \qquad (14)$$

$$= N\psi(\Omega,t) + D\nabla^2 \psi(\Omega,t) \qquad (15)$$

where are defined the normalization and diffusion coefficients as:

$$N = \int_{S^2} W_{hom}(\overline{\Omega}) d\overline{\Omega} \qquad (16)$$

$$D = \frac{1}{4} \int_{S^2} \bar{\theta}^2 W_{hom}(\overline{\Omega}) d\overline{\Omega} \qquad (17)$$

$$(18)$$

These quantities can be computed from the equation of the local kernel. Considering two examples:

Gaussian kernel

The default local kernel in TVB is Gaussian:

$$Ae^{-\frac{(x-m)^2}{2\sigma^2}} + k \qquad (19)$$

with default values A = 1, $\sigma$ = 1, m = 0 and k = 0 it becomes simply:

$$e^{-\frac{x^2}{2}} \qquad (20)$$

Thus, the diffusion approximation for the default homogeneous kernel is:

$$N = r^2 \int_0^{2\pi} \int_0^{\pi} e^{-\frac{(r\bar\theta)^2}{2}} \sin(\bar\theta) \, d\bar\theta d\bar\phi \simeq 6.28298 \text{ for } r=100 \text{ mm}$$

$$D = \frac{1}{4} r^2 \int_0^{2\pi} \int_0^{\pi} \bar\theta^2 e^{-\frac{(r\bar\theta)^2}{2}} \sin(\bar\theta) \, d\bar\theta d\bar\phi \simeq 0.000314138 \text{ for } r=100 \text{ mm}$$

Laplacian kernel

The local kernel typically used for the neural field simulations using the Epileptor model is Laplacian:

$$\frac{A}{2b} e^{-\frac{|x|}{b}} + k \tag{21}$$

with default values A = 1, b = 1 and k = 0 it becomes simply:

$$\frac{1}{2} e^{-|x|}$$

Thus, the diffusion approximation for the Laplacian homogeneous kernel is:

$$N = r^2 \int_0^{2\pi} \int_0^{\pi} \frac{1}{2} e^{-r\bar\theta} \sin(\bar\theta) \, d\bar\theta d\bar\phi = \frac{\pi(e^{-\pi r} + 1)r^2}{r^2 + 1} \simeq 3.14128 \text{ for } r=100 \text{ mm}$$

$$D = \frac{1}{4} r^2 \int_0^{2\pi} \int_0^{\pi} \bar\theta^2 \frac{1}{2} e^{-r\bar\theta} \sin(\bar\theta) \, d\bar\theta d\bar\phi = \frac{\pi e^{-\pi r} r^2 (4\pi(r^3 + r) + 6r^2 + \pi^2(r^2 + 1)^2 + e^{\pi r}(6r^2 - 2) - 2)}{4(r^2 + 1)^3} \simeq$$
$$\simeq 0.00047108 \text{ for } r=100 \text{ mm}$$

Using the approximated equation (15), we can see that the convolution integral in equation (11) has been approximated with a Laplacian operator. While this approximation is not necessary for the pseudo-spectral approach, it simplifies calculations and results. Calling FFT the projection of the state variable onto the spherical harmonics base, the integral becomes (here the linear term is not inserted, being of trivial computation):

$$I \simeq \text{FFT}^{-1}\left(-Dl(l+1) \cdot \text{FFT}\left(\psi(\Omega, t)\right)\right) \tag{22}$$

To get a quantitative estimation of the expected speed up that the mode decomposition technique should provide, a benchmark of the implementation to compute equations (12) and (22) has been performed. Notice that the two equations are approximating two different models of the homogeneous connectivity, that are related through the approximation derived in equation (15). The results are shown in Fig. 8. For a benchmark of the local coupling term in isolation, refer to the local coupling term benchmark explained below.

Use case: Generic 2D oscillator

To validate and benchmark the pseudo-spectral implementation in TVB we have considered a simple model, called the generic 2D oscillator; this model can reproduce various dynamical features depending on the choice of its parameters. It could be used to investigate resting state activity or the effects of a tumour on whole brain dynamics. To illustrate the invention, the same analysis could be easily reproduced with any other model. The parameters of the model have been set so that the dynamics of the isolated neural population would fall into a limit cycle; a Gaussian function has been used for the homogeneous kernel, thus the diffusion coefficient has been set as computed in equation (20).

To evaluate the approximation introduced by our technique we have computed the mode level cognitive subtraction (MLCS, see reference document [1]) between the pseudo-spectral results and the neural field obtained computing the Laplace-Beltrami operator (more details in the Laplace-Beltrami operator explained below). Both the simulations are numerical approximations of equation (15). The resulting spatiotemporal evolutions are represented in Fig. 9.

Benchmarking

To evaluate the speed up obtained with the pseudo-spectral approach, a sweep of simulations over meshes with different resolution of the same patient has been run, both for TVB and the pseudo-spectral simulator.

The cut-off has been set to 10 mm, and the truncation mode has been chosen to be L = 70. The results are represented in Fig. 13. The pseudo-spectral approach performs, in the range of interest, at least an order of magnitude better than the TVB simulator.

Use case: epileptic seizure

Using the Epileptor neural field as in reference document [6], we simulated a seizure using the data of a human patient. The epileptic zones, with labels *ctx-lh-temporalpole, Left-Hippocampus,* have been set to an excitability of -1.8, while the propagation zones, with labels *Left-Rhinal-cortex, Left-ITS-anterior, Left-STS-anterior* and *Left-T2-anterior* have been set to an excitability of -2, and the remaining regions, including the whole right hemisphere, has been set to an excitability of -2.3. With these settings, no appreciable evolution happens in the right hemisphere; thus, in the visualization we will only show the left hemisphere.

The full resolution mesh obtained with Freesurfer™ has been used, thus a mesh of 266887 vertices represents the cortical surface. In the TVB simulation, the cut-off has been set to 10 mm, that is adequate considering the fast decay of the Laplacian kernel (21). In the pseudo-spectral simulation, the truncation of the modes has been set to L = 250, the maximum possible with this grid resolution.

The diffusion coefficient has been set according to the Laplace kernel (equation (21)), that is the kernel used in reference document [6]. The spatiotemporal evolution of the left hemisphere and the quantitative matching between the pseudo-spectral and the TVB simulations are shown in Fig. 10; notice that here we are visually comparing the results obtained simulating 1 with TVB and equation 15 with the pseudo-spectral simulator.

The simulated seizure lasts 10 seconds; with the pseudo-spectral method the whole simulation took 2700 seconds, while using TVB it took 70000 seconds. The local coupling evaluation took 3.2 ms per iteration in the pseudo-spectral case, while in The Virtual Brain method according to the state of the art, it required 216 ms per iteration. While the pseudo-spectral results are good enough for many application purposes, it is evident, both from the MLCS and the spatiotemporal representation, that some dynamical features are lost; there are many sources of error in this use case, and there is room for future improvement. To mention one: in the neural field Epileptor model of reference document [6] the local coupling is a highly discontinuous term in space (the step function is applied the $\psi$); the FFT applied to a discontinuous function needs extra-care to be evaluated.

High resolution simulations allow for a better fitting of the brain zones, such as the epileptic zone in the epilepsy context. The high resolution comes at the cost of a high dimensionality: for every vertex of the mesh a value of the excitability has to be determined, and there is no available model inversion technique that can manage such a high dimensional parameter space.

The mode decomposition can be applied to reduce such dimensionality: instead of identifying the excitabilities vertex by vertex, we can decompose the excitability as previously described for the activity into spherical harmonics on the sphere, identify the weights and then reconstruct the spatial distribution to run the simulation. With this approach, the number of parameters to be identified can be reduced to a few hundred/thousands, the precise number of which depends on the degree of approximation required by the task at hand.

This application of the invention is not restricted to the epilepsy field, it can be used every time the dimensionality of a spatially extended function needs to be reduced; the same procedure could be applied for the subnetwork in the case of Parkinson disease.

Conclusions

As a conclusion, the invention concerns a new method to efficiently simulate neural field equations. To summarize:

- the folded cortical surface is inflated to a sphere, using pre-existent routines that minimize metrical distortions.

- the simulation happens on the spherical surface, where a Fourier transform technique allows to greatly reduce the computational expensiveness trading accuracy.
- the resulting neural field values are mapped back to the folded surface to obtain the source signal.

The results and the benchmark of three possible implementations of the algorithm have been shown, namely:

when analytical treatment is possible, a spectral approach maps the solution of the integro-PDEs into a tunable number of delayed differential equations (DDEs). The resulting simulator performance does not depend on the resolution of the mesh.

practically, non-linear terms in the neural field equations require numerical methods to perform the Fourier transform: we have illustrated a simulator that uses a semi-implicit integrator and the FFT algorithm on a spherical domain, and we have compared it to an in-house implementation of the finite volume method.

- to compare the mode decomposition approach performances to state-of-the-art software, we have introduced the pseudo-spectral method into the TVB framework, and we have compared its results with TVB in two use cases: an oscillatory activity using the generic 2D oscillator and an epileptic seizure.

All of the implementations share the same conclusions: the mode decomposition allows to trade accuracy with time consumption, and scales much better than the current methods increasing the resolution of the cortical mesh. Hence, Due to the high spatial density of the number of neurons in the cerebral cortex, it is possible to represent the mean neural activity as a continuous function of space; neural field models relevance resides in their link with experimental data obtained through neural recording techniques, such as electroencephalogram (EEG) and functional magnetic resonance imaging (fMRI). Typically, neural eld models contain integro-differential equations that involve a term describing the connectivity between neural populations.

The high-resolution data that is available nowadays requires efficient numerical methods to be included into simulations of neural field equations. The mesh that represents the cortical surface needs a good resolution in order to correctly represent the neural sheet, and the computation of the connectivity terms poses memory and time challenges. The homogeneous connectivity term, that represents the connections among nearby neural populations, is typically the bottleneck of these simulations.

Thus, mathematical methods to reduce the computational burden of such simulations are needed. Here, the mode decomposition method is presented: the folded neural sheet is inflated to a sphere using the FreeSurfer™ software, then, to optimize the computation of the value at the next time step, the neural field is decomposed into modes using the Fourier transform, and then the modes are recomposed using the inverse Fourier transform to reconstruct the solution on the physical space. The neural field is then projected back to the folded surface representing the cortex, to obtain the source signal. An analysis of the approximations introduced with this method is presented here.

Here we show that using a pseudo-spectral approach it is possible to obtain a great speed up for high-resolution meshes. The computational efficiency of the method comes at a price: spatial deformations are introduced, and only the contribution of some of the spatial modes that compose the neural field is considered in the spatiotemporal evolution. The number of spatial modes to consider can be tuned setting the parameter L and is only limited by the resolution of the data. This induces an artificial resolution limit that scales with $1/L^2$.

The Laplace-Beltrami operator

To compare the results obtained with the pseudo-spectral simulator without any approximation in the local coupling term, we have implemented in TVB a numerical scheme to directly evaluate equation 15. The Laplace operator is estimated using the heat kernel; the solution to the heat equation:

$$\triangle_S u(x,t) = \frac{\partial u}{\partial t}(x,t)$$

$$u(x,0) = f(x)$$

can be written:

$$u(x,t) = \int_S H_S^t(x,y) f(y) d\nu(y) \qquad (23)$$

Substituting this back to the heat equation and taking the limit for t that goes to 0, in equation [2] it has been proved that the discrete Laplace-Beltrami operator $L_K^h f(w)$ can be approximated as:

$$L_K^h f(w) = \frac{1}{4\pi h^2} \sum_{t \in K} \frac{\text{Area}(t)}{\#t} \sum_{p \in V(t)} e^{-\frac{|p-w|^2}{4h}} (f(p) - f(w)) \qquad (24)$$

We used this result to evaluate in space equation (15).

Local coupling term benchmark

The performance of the implementation in TVB of equation (12) depends on the degree of sparsity of the matrix representing the homogeneous kernel, that is parameterized through a cut-off value (namely the maximum length of a local coupling connection); a sweep over different cut-off values with a fixed mesh is represented in Fig. 11. For every cut-off value, a TVB simulation with 300000 evaluations of the local coupling term has been run; then the total time spent inside the function that performs the matrix vector multiplication in equation (12) has been divided by the number of evaluations. Same numbers have been used for the rest of the benchmarking results in this section. Using the full mesh, we performed a benchmarking only with a cut-off of 10 mm, and the time consumption per evaluation has been evaluated as 112 ms. We did not sweep over higher values of the cut-off, as the time and the memory requirements became restrictive.

The pseudo-spectral simulation's performance depends on the required accuracy, that can be set through the number of spatial modes that are used to represent the neural field values in the mode space. In Fig. 12, a sweep over different truncation values L is carried on (the total number of modes is thus obtainable as L(L - 1)=2).

Hence, the neural field simulator proposed in this invention accomplishes an acceleration in simulation speed and is based on dimensionality reduction techniques that can be applied for model inversion.

Finally, the invention has a wide range of applications, in particular virtual brain simulations, in which scientific or clinical questions are tackled, such as:

- Epilepsy: building a patient-specific virtual brain allows to identify the excitability spatial distribution through model inversion (for example, via simulation-based inference (SBI)); the number of parameters would match the number of neural populations, that for high-resolution data would render nowadays techniques computationally unfeasible. To reduce the dimensionality of the parameter space, the same spectral decomposition used for the simulator of the invention can be applied to the excitability distribution, so that the number of parameters to be identified can be reduced to a few hundred, and current model inversions techniques can be applied.
- Tumor effects on brain functions: a brain tumor affects the structure and the activity of the brain. The neural field simulator allows investigating at high-resolution the effects of the perturbation induced by a tumor on brain activity.
- Brain stimulation: the neural field simulator of this invention allows investigating the effects of stimulations such as deep brain stimulation (DBS) and transcranial stimulation with direct/alternate current stimulation (tDCS or tACS) at high-resolution, at the whole-brain level. The electric fields would be represented in a biologically realistic matter, as millimeter resolution is required to define a local current direction meaningfully; realistic traveling waves could be reproduced and investigated.

The reference documents cited above are as follows:

[1] Arpan, Banerjee; Emmanuelle, T. C. A. S. K. V. J. Mode level cognitive subtraction (mlcs) quanties spatiotemporal reorganization in large-scale brain topographies. NeuroImage 42 (2008) 663-674.

[2] Belkin, Mikhail; Sun, J. W. Y. Discrete Laplace operator on meshed surfaces.

[3] Daini, D., Ceccarelli, G., Cataldo, E., and Jirsa, V. Spherical-harmonics mode decomposition of neural field equations. Phys. Rev. E 101 (Jan 2020), 012202.

[4] Ju, L., and Du, Q. A finite volume method on general surfaces and its error estimates. Journal of Mathematical Analysis and Applications 352, 2 (2009), 645{668.

[5] Schaeffer, N. Efficient spherical harmonic transforms aimed at pseudospectral numerical simulations. Geochemistry, Geophysics, Geosystems 14 (03 2013).

[6] Timothee, Proix; Viktor, J. F. B. M. G. W. T. Predicting the spatiotemporal diversity of seizure propagation and termination in human focal epilepsy. Nature Communications 9 (12 2018).

[7] Wieczorek, Mark A.; Meschede, M. Shtools - tools for working with spherical harmonics. Geo-chemistry, Geophysics, Geosystems (05 2018).

**Claims**

1. A method of simulating a human brain neural field in a computerized platform modelling various zones of a human brain, comprising the following steps:

   providing the computerized platform modelling the various zones of the human brain;
   acquiring three-dimensional anatomical structural imaging data of a folded surface of a cortex of a brain of a human patient;
   personalizing the computerized platform according to the structural data;
   providing an equation describing a spatiotemporal evolution of the neural field and loading the equation in the computerized platform;
   performing a transformation of the surface of the cortex of the brain of the patient to a spherical surface domain;
   simulating the neural field in the spherical surface domain; and
   translating the simulated neural field obtained in the spherical domain to the cortical domain.

2. The method of claim 1, wherein the three-dimensional anatomical structural imaging data of the folded surface of the cortex of the brain of the human patient are Magnetic Resonance Imaging data.

3. The method of claim 2, wherein the Magnetic Resonance Imaging Data include Diffusion or Functional Magnetic Resonance Imaging Data.

4. The method of one of the claims 1, 2 or 3, wherein the computerized platform is modelling various zones of the human brain and connectivity between said zones.

5. The method of one of the previous claims, wherein the simulation of the neural field in the spherical domain is decomposed into modes using a Fourier transform, and the modes are recomposed using an inverse Fourier transform.

6. The method of one of the previous claims, wherein the equation describing the spatiotemporal evolution of the neural field is:

$$\frac{\partial \psi(\Omega_1, t)}{\partial t} = \mathcal{N}(\psi(\Omega_1, t)) +$$

$$+ \int_\Gamma W_{hom}(d_g(\Omega_1, \Omega_2)) S[\psi(\Omega_2, t - d_g(\Omega_1, \Omega_2)/c)] d\Omega_2 +$$

$$+ \int_\Gamma W_{het}(\Omega_1, \Omega_2) S[\psi(\Omega_2, t - d(\Omega_1, \Omega_2)/v)] d\Omega_2$$

wherein

$\Omega$ represents the coordinates of a point on the surfacer $\Gamma$ $\psi(\Omega, t)$ is a vector that contains the state variables of

the model,

$W_{hom}(d_g(\Omega_1, \Omega_2))$ is a homogeneous kernel, a function of the geodesic distance between two points that defines a strength and a sign of the local connection,

$W_{het}(\Omega_1, \Omega_2)$ is a heterogeneous kernel, a function that defines if and how any couple of points of the surface is connected through a myelinated connection,

$d(\Omega_1, \Omega_2)$ represents a length of a fibre connecting the two points.

7. The method of claim 6, wherein, assuming that homogenous connectivity is short-ranged, and that heterogeneous connections are pointwise, the equation describing the spatiotemporal of the neural field is approximated as

$$\frac{\partial \psi(\Omega_1, t)}{\partial t} = -\epsilon \psi(\Omega_1, t) + D\nabla^2 \psi(\Omega_1, t) + \sum_{i,j=1}^{N} \mu_{ij}\delta(\Omega_1 - \Omega_i)S\left(\psi\left(\Omega_j, t - \frac{d}{v}\right)\right)$$

8. The method of one of the previous claims, wherein the equation describing the spatiotemporal evolution of the neural field is linear.

9. The method of one of claims 1, 2 or 3, wherein the equation describing the spatiotemporal evolution of the neural field comprises a non-linear term.

10. The method of one of the previous claims, wherein the equation describing a spatiotemporal evolution of the neural field is calculated using a finite volume method.

11. The method according to the invention, wherein the surface of the cortex of the brain of the patient is tessellated according to a mesh of at least 10000 vertices.

12. The method of one of the previous claims, wherein the human brain is an epileptic human brain, and wherein the simulated neural field is the neural field of the epileptic human brain during an epileptic seizure.

13. The method of one of the previous claims, wherein the human brain is a human brain including a tumour, and wherein effects of the tumour on a structure and/or an activity of the human brain are simulated.

14. The method of one of the previous claims, wherein effects of stimulation, such as deep brain stimulation or transcranial stimulation are simulated.

15. The method of one of the previous claims, wherein the human brain is an Alzheimer human brain, and wherein the simulated neural field is the neural field of the Alzheimer human brain.

16. A Simulator of a human brain neural field in a computerized platform modelling various zones of a human brain, comprising:

the computerized platform modelling the various zones of the human brain;
three-dimensional anatomical structural imaging data of a folded surface of a cortex of a brain of a human patient;
the computerized platform being personalized according to the structural data;
an equation describing a spatiotemporal evolution of the neural field, the equation being loaded in the computerized platform;
computing means for performing a transformation of the surface of the cortex of the brain of the patient to a spherical surface domain;
simulating means for simulating the neural field in the spherical surface domain; and
translating means for translating the simulated neural field obtained in the spherical domain to the cortical domain.

Fig. 1A

Fig. 1B

Fig. 2

Fig. 3

Fig. 4

Fig. 5A

Fig. 5B

Fig. 6A

Fig. 6B

Fig. 7

## Mode decomposition

Fig. 8A

## TVB simulation

Fig. 8B

Fig. 9A

Fig. 9B

Fig. 9C

Fig. 10A

Fig. 10B

Fig. 10C

Fig. 11

Fig. 12

Fig. 13

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SPIEGLER A ET AL: "Systematic approximations of neural fields through networks of neural masses in the virtual brain", NEUROIMAGE, vol. 83, 15 June 2013 (2013-06-15), pages 704-725, XP028758262, ISSN: 1053-8119, DOI: 10.1016/J.NEUROIMAGE.2013.06.018 | 1-3,5, 7-10,13, 14,16 | INV. G16H50/50 |
| Y | * page 704, column 1, paragraph 1 - page 705, column 1 * <br> * page 704, column 2 - page 705, column 1 * <br> * page 707, column 1 * <br> ----- | 4,6,11, 12,15 | |
| Y | BLAKE J COOK ET AL: "Neural Field Models: historical perspectives and recent advances", ARXIV.ORG, CORNELL UNIVERSITY LIBRARY, 201 OLIN LIBRARY CORNELL UNIVERSITY ITHACA, NY 14853, 18 March 2021 (2021-03-18), XP081913398, * page 1, paragraph 1 - page 2, paragraph 2 * <br> * page 26 * <br> * page 43 * <br> ----- | 12,15 | |
| Y | KUNZE TIM ET AL: "Transcranial direct current stimulation changes resting state functional connectivity: A large-scale brain network modeling study", NEUROIMAGE, ELSEVIER, AMSTERDAM, NL, vol. 140, 13 February 2016 (2016-02-13), pages 174-187, XP029723964, ISSN: 1053-8119, DOI: 10.1016/J.NEUROIMAGE.2016.02.015 * page 175, column 2 * <br> ----- | 6 | |

TECHNICAL FIELDS
SEARCHED (IPC)

G16H

-/--

The present search report has been drawn up for all claims

| Place of search | Date of completion of the search | Examiner |
|---|---|---|
| Munich | 6 May 2022 | Laub, Christoph |

EPO FORM 1503 03.82 (P04C01)

page 1 of 3

Europäisches
Patentamt

European
Patent Office

Office européen
des brevets

## EUROPEAN SEARCH REPORT

Application Number

EP 21 21 0045

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JIRSA V K ET AL: "SPATIOTEMPORAL FORWARD SOLUTION OF THE EEG AND MEG USING NETWORK MODELING", IEEE TRANSACTIONS ON MEDICAL IMAGING, IEEE, USA, vol. 21, no. 5, 1 May 2002 (2002-05-01), pages 493-504, XP001114962, ISSN: 0278-0062, DOI: 10.1109/TMI.2002.1009385 * page 496, paragraph III * * page 499, paragraph E * * page 1, paragraph 2 * | 4 | |
| A | PAUL C BRESSLOFF: "Topical Review;Spatiotemporal dynamics of continuum neural fields;Spatiotemporal dynamics of continuum neural fields", JOURNAL OF PHYSICS A: MATHEMATICAL AND THEORETICAL, INSTITUTE OF PHYSICS PUBLISHING, BRISTOL, GB, vol. 45, no. 3, 14 December 2011 (2011-12-14), page 33001, XP020217241, ISSN: 1751-8121, DOI: 10.1088/1751-8113/45/3/033001 * the whole document * | 1-16 | |
| A | PINOTSIS D A ET AL: "Neural fields, spectral responses and lateral connections", NEUROIMAGE, ELSEVIER, AMSTERDAM, NL, vol. 55, no. 1, 26 November 2010 (2010-11-26), pages 39-48, XP028171473, ISSN: 1053-8119, DOI: 10.1016/J.NEUROIMAGE.2010.11.081 [retrieved on 2010-12-04] * page 40 – page 41 * | 1-16 | |

TECHNICAL FIELDS SEARCHED (IPC)

-/--

The present search report has been drawn up for all claims

| Place of search | Date of completion of the search | Examiner |
|---|---|---|
| Munich | 6 May 2022 | Laub, Christoph |

EPO FORM 1503 03.82 (P04C01)

page 2 of 3

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | "Modeling & analysis ED – Johansen-Berg Heidi; Duzel Emrah", NEUROIMAGE, ELSEVIER, AMSTERDAM, NL, vol. 16, no. 2, 1 January 2002 (2002-01-01), pages 769-1198, XP026928524, ISSN: 1053-8119, DOI: 10.1016/S1053-8119(02)90013-3 [retrieved on 2002-01-01] * page 139 * ----- | 11 | |
| | | | **TECHNICAL FIELDS SEARCHED (IPC)** |

The present search report has been drawn up for all claims

| Place of search | Date of completion of the search | Examiner |
|---|---|---|
| Munich | 6 May 2022 | Laub, Christoph |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................
& : member of the same patent family, corresponding document

EPO FORM 1503 03.82 (P04C01)

**page 3 of 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018015778 A

### Non-patent literature cited in the description

- **JIRSA et al.** On the nature of seizure dynamics. *Brain,* 2014, vol. 137, 2210-2230
- **TIMOTHEE PROIX et al.** Permittivity Coupling across Brain Regions Determines Seizure Recruitment in Partial Epilepsy. *The Journal of Neuroscience,* 05 November 2014, vol. 34 (45), 15009-15021
- **ARPAN, BANERJEE ; EMMANUELLE, T. C. A. S. K. V. J.** Mode level cognitive subtraction (mlcs) quanties spatiotemporal reorganization in large-scale brain topographies. *NeuroImage,* 2008, vol. 42, 663-674
- **BELKIN, MIKHAIL ; SUN, J. W. Y.** *Discrete Laplace operator on meshed surfaces*
- **DAINI, D. ; CECCARELLI, G. ; CATALDO, E. ; JIRSA, V.** Spherical-harmonics mode decomposition of neural field equations. *Phys. Rev. E,* January 2020, vol. 101, 012202
- **JU, L. ; DU, Q.** A finite volume method on general surfaces and its error estimates. *Journal of Mathematical Analysis and Applications,* 2009, vol. 352 (2
- **SCHAEFFER, N.** Efficient spherical harmonic transforms aimed at pseudospectral numerical simulations. *Geochemistry, Geophysics, Geosystems,* March 2013, vol. 14
- **TIMOTHEE, PROIX ; VIKTOR, J. F. B. M. G. W. T.** Predicting the spatiotemporal diversity of seizure propagation and termination in human focal epilepsy. *Nature Communications,* December 2018, vol. 9
- **WIECZOREK, MARK A. ; MESCHEDE, M.** Shtools - tools for working with spherical harmonics. *Geo-chemistry, Geophysics, Geosystems,* May 2018